# EUROPEAN PATENT SPECIFICATION

(11) **EP 1 968 506 B1**
(45) Date of publication and mention of the grant of the patent: **14.09.2011**
(21) Application number: 06845988.2
(22) Date of filing: 21.12.2006
(51) Int. Cl.: A61F 5/00

(54) **COILED INTRAGASTRIC MEMBER FOR TREATING OBESITY**
AUFGEROLLTES INTRAGASTRISCHES ELEMENT ZUR BEHANDLUNG VON ADIPOSITAS
MEMBRE INTRAGASTRIQUE EN SPIRALE POUR LE TRAITEMENT DE L'OBESITE

(30) Priority: 22.12.2005 US 753252 P
(43) Date of publication of application: 17.09.2008
(73) Proprietor: Wilson-Cook Medical Inc., Winston-Salem, NC 27105-4191 (US); Cook Ireland Ltd, Limerick (IE)
(72) Inventor: SKERVEN, Gregory, J., Kernersville, NC 27284 (US); SOETERMANS, Max, Pinnacle, NC 27043 (US); O'SULLIVAN, Donagh, Castleroy, Limerick (IE)
(74) Representative: Garratt, Peter Douglas
(86) International application number: PCT/US2006/048957
(87) International publication number: WO 2007/075978

(56) References cited:
- WO-A-88/00027
- WO-A-90/00369
- WO-A-02/091961
- US-A1- 2003 109 935
- US-A1- 2005 267 596

## Description

### TECHNICAL FIELD

This invention relates to medical devices, and more particularly to obesity treatment devices that can be placed in the stomach of a patient to reduce the size of the stomach reservoir or to place pressure on the inside surface of the stomach.

### BACKGROUND OF THE INVENTION

It is well known that obesity is a very difficult condition to treat. Methods of treatment are varied, and include drugs, behavior therapy, and physical exercise, or often a combinational approach involving two or more of these methods. Unfortunately, results are seldom long term, with many patients eventually returning to their original weight over time. For that reason, obesity, particularly morbid obesity, is often considered an incurable condition. More invasive approaches have been available which have yielded good results in many patients. These include surgical options such as bypass operations or gastroplasty. However, these procedures carry high risks and are therefore not appropriate for most patients.

In the early 1980s, physicians began to experiment with the placement of intragastric balloons to reduce the size of the stomach reservoir, and consequently its capacity for food. Once deployed in the stomach, the balloon helps to trigger a sensation of fullness and a decreased feeling of hunger. These balloons are typically cylindrical or pear-shaped, generally range in size from 200-500 ml or more, are made of an elastomer such as silicone, polyurethane, or latex, and are filled with air, water, or saline. While some studies demonstrated modest weight loss, the effects of these balloons often diminished after three or four weeks, possibly due to the gradual distension of the stomach or the fact that the body adjusted to the presence of the balloon. Other balloons include a tube exiting the nasal passage that allows the balloon to be periodically deflated and re-insufflated to better simulate normal food intake. However, the disadvantages of having an inflation tube exiting the nose are obvious.

The experience with balloons as a method of treating obesity has provided uncertain results, and has been frequently disappointing. Some trials failed to show significant weight loss over a placebo, or were ineffective unless the balloon placement procedure was combined with a low-calorie diet. Complications have also been observed, such as gastric ulcers, especially with use of fluid-filled balloons, and small bowel obstructions caused by deflated balloons. In addition, there have been documented instances of the balloon blocking off or lodging in the opening to the duodenum, wherein the balloon may act like a ball valve to prevent the stomach contents from emptying into the intestines.

Unrelated to the above-discussed methods for treating obesity, it has been observed that the ingestion of certain indigestible matter, such as fibers, hair, fuzzy materials, etc., can collect in the stomach over time, and eventually form a mass called a bezoar. In some patients, particularly children and the mentally handicapped, bezoars often result from the ingestion of plastic or synthetic materials. In many cases, bezoars can cause indigestion, stomach upset, or vomiting, especially if allowed to grow sufficiently large. It has also been documented that certain individuals having bezoars are subject to weight loss, presumably due to the decrease in the size of the stomach reservoir. Although bezoars may be removed endoscopically, especially in conjunction with a device known as a bezotome or bezotriptor, they, particularly larger ones, often require surgery.

An artificial bezoar is disclosed in WO-A-02/091961.

What is needed is an intragastric member that is easily delivered to the stomach of a patient to reduce the size of the stomach while also applying pressure on the inside surface of the stomach to create a feeling of fullness.

### SUMMARY OF THE INVENTION

The foregoing problems are solved and a technical advance is achieved by an illustrative obesity treatment apparatus comprising at least one intragastric member comprising a curvilinear axis or artificial bezoar made of a digestive-resistant or substantially indigestible material that is introduced into a the gastric lumen of a mammal in a first configuration. The intragastric member or artificial bezoar is typically inserted into the gastric lumen in a partially compacted configuration, whereby it is then manipulated into, or allowed to assume, a second expanded configuration sufficiently large to remain within the reservoir of the stomach during normal activities and not be passed through the pylorus and into the intestines. The present invention can also be effective at a smaller volume within the stomach than existing intragastric members, such as balloons.

In one aspect of the invention, the obesity treatment apparatus comprises an intragastric member expandable from a first configuration to a second configuration, the first configuration being sufficiently small to permit introduction of said intragastric member into a gastric lumen of a mammal, the second configuration being sufficiently large to prevent said intragastric device from passing through the mammal's pylorus.

In another aspect of the invention, the obesity treatment apparatus comprises an intragastric member comprising a curvilinear axis which extends about and along a central axis of an intragastric device. The curvilinear axis of the intragastric member is spaced away from the central axis by a predetermined distance or a variable distance. The intragastric member comprises a shape selected from one of a spiral, helix, coil, cork screw, spring and loop.

In another aspect of the invention, the obesity treatment apparatus comprises an intragastric member including a proximal end, a distal end and a lumen extending between the proximal end and the distal end, wherein the lumen is utilized to inflate the intragastric member to the second configuration. The intragastric member can also comprise an opening in communication with the lumen, wherein the opening is utilized to inflate the lumen of the intragastric member with pressurized gas or liquid. In an alternate embodiment, the intragastric member can include a self-expanding metal, such as nitinol.

In another aspect, the obesity treatment device includes a delivery system to place the intragastric member within the gastric lumen. In one embodiment, one or more intragastric members are mounted on a delivery tube and secured with a releasing mechanism, such as a nylon thread, extending through the passageway of the delivery tube. A metal wire or loop is then withdrawn, severing the threads and releasing the intragastric member(s) into the gastric lumen. The individual intragastric members are then secured with a device such as a rubber patch pushed by an introduced metal tube or similar device.

In yet another aspect of the invention, the obesity treatment apparatus can comprise a plurality of intragastric members that are secured with a releasing mechanism, wherein the plurality of intragastric members are secured in the first configuration by the releasing mechanism then released in the gastric lumen. Other delivery systems of the present invention can include pushing the intragastric member(s) from an outer delivery catheter, typically by use of pusher member within the delivery catheter passageway. Other methods include constraining the intragastric member(s) with a splittable or dissolvable film or sheath that allows that device to be deployed in a compact configuration, then allowing intragastric member to expand when the outer wrapping or sheath is split by the operator.

In yet another aspect of the invention, the obesity treatment apparatus can comprise an intragastric member comprising one or more elongate portions inflatable from a first configuration to a second configuration, wherein the one or more elongate portions comprise a lumen extending through a portion thereof, wherein the lumen is inflated with a material to provide rigidity to the intragastric member.

In still yet another aspect of the invention, the obesity treatment apparatus can comprise one or more intragastric members made of a preformed spiral coil loaded onto a delivery tube in a partially compacted first configuration, wherein the assembly is delivered through a flexible overtube. The flexible overtube includes a proximal end, a distal end, and a lumen configured to receive the intragastric members in the first configuration for delivery to the gastric lumen wherein the digestive-resistant material of the intragastric member is expanded to a second configuration when in the gastric lumen.

In yet another aspect, a method of treatment of obesity in mammals comprises the steps of providing a delivery tube comprising a lumen, a proximal end and a distal end and loading at least one intragastric member between the proximal end and the distal end of the delivery tube, wherein the intragastric member comprises a preformed spiral coil compacted into a first configuration that is sufficiently small to permit introduction into the gastric lumen of mammal. The method also includes the steps of positioning the delivery tube comprising the intragastric member within a lumen of a flexible overtube and advancing the intragastric member through the lumen of the flexible overtube into the gastric lumen of the mammal. The method further includes the step of expanding the intragastric member into a second configuration that is sufficiently large to prevent the intragastric member from passing the mammal's pylorus.

These and other advantages, as well as the invention itself, will become apparent in the details of construction and operation as more fully described below. Moreover, it should be appreciated that several aspects of the invention can be used with other types of intragastric devices or procedures used for the treatment of obesity.

### BRIEF DESCRIPTION OF SEVERAL VIEWS OF THE DRAWINGS

Several embodiments of the present invention will now be described by way of example with reference to the accompanying drawings, in which:

**FIG. 1** depicts a pictorial view of an intragastric member of the present invention;

**FIG. 2** depicts a pictorial view of a pair of intragastric members of the present invention after being coupled together,

**FIG. 3** depicts a pictorial view of the embodiment of FIG. 1 with a delivery system;

**FIG. 4** depicts a sectional view of the delivery system of FIG. 3;

**FIG. 5** depicts an intragastric member loaded onto a delivery tube for insertion into the gastric lumen;

**FIG. 6** depicts an intragastric member of the present invention in a first configuration with retaining element after delivery to the gastric lumen;

**FIG. 7** depicts a self expanding intragastric member of the present invention after delivery to the gastric lumen;

**FIG. 8** depicts an inflatable intragastric member after delivery to the gastric lumen;

**FIG. 9** depicts yet another embodiment of a self-expanding intragastric member of the present invention after delivery to the gastric lumen;

**FIG. 10** depicts yet another embodiment of an inflatable intragastric member after delivery to the gastric lumen;

**FIG. 11** depicts a pictorial view of another embodiment of an intragastric member of the present invention;

**FIG. 12** depicts a pictorial view of the intragastric member of FIG. 11 in an expanded second configuration;

**FIG. 13** depicts a pictorial view of the intragastric member of FIG. 11 in a first configuration after delivery to the gastric lumen;

**FIG. 14** depicts the intragastric member of FIG. 13 in an expanded second configuration after delivery to the gastric lumen;

**FIG. 15** depicts a pictorial view of yet another embodiment of an intragastric member of the present invention;

**FIG. 16** depicts the intragastric member of FIG. 15 in an expanded second configuration after delivery to the gastric lumen;

**FIG. 17** depicts a pictorial view of yet another embodiment of an intragastric member of the present invention;

**FIG. 18** depicts the intragastric member of FIG. 17 in an expanded second configuration;

**FIG. 19** depicts a pictorial view of the embodiment of FIG. 17 in a first configuration after delivery to the gastric lumen;

**FIG. 20** depicts the intragastric member of FIG. 17 in a second configuration after delivery to the gastric lumen; and

**FIG. 21** depicts a partial, cross-sectional view showing an overtube positioned in the mouth and along the esophagus of a patient such that the overtube distal end is positioned in the gastric lumen of the stomach.

### DETAILED DESCRIPTION OF THE INVENTION

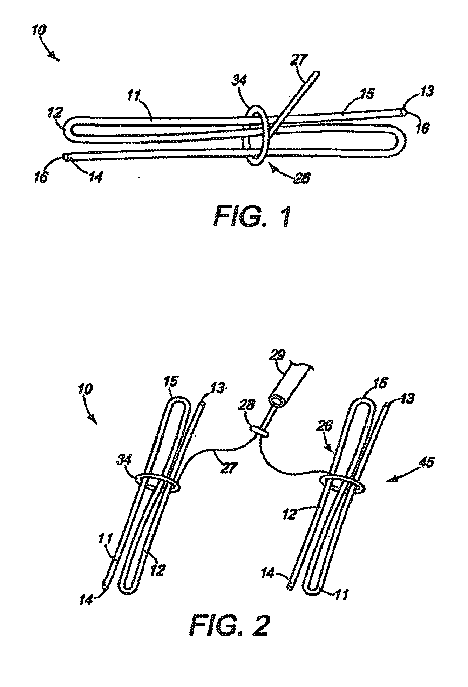

The obesity treatment apparatus **10** of the present invention depicted in **FIGS. 1-21** comprise one or more intragastric members **11,** each comprising a curvilinear axis forming a preformed spiral coil **15** sized and configured such that the intragastric member **11** can be delivered to the stomach of a mammalian patient and reside therein, without passing through the pylorus. As used herein, the terms digestive-resistant and indigestible are intended to mean that the material used is not subject to the degrative effects of stomach acid and enzymes, or the general environment found within the gastric system over an extended period of time, therefore allowing the device to remain intact for the intended life of the device. This does not necessarily mean that the material cannot be degraded over time; however, one skilled in medical arts and gastrological devices would readily appreciate the range of material that would be suitable for use as a long-term intragastric member.

Many well-known plastics have suitable properties, including selected polyesters, polyurethanes, polyethylenes, polyamides, silicone, or other possible materials. Mammalian hair has been found to form natural bezoars, and thus, is also a possible material. However, some materials, such as certain polyamides, have been found to expand over time, which can be an undesirable property. Most other natural materials are generally much less resistant to acids and enzymes, and would therefore typically require treatment or combination with resistant materials to function long term, unless a shorter-term placement is intended or desired.

Additionally, the intragastric member **11** may be formed from a shape memory material, such as nitinol. Additionally, the shape memory material may comprise a polymer material capable of retaining a predetermined shape using heat-treatment techniques. The intragastric member **11** may be heated to a temperature exceeding the glass temperature of the polymer and shaped into a predetermined configuration. The intragastric member **11,** when implanted within the body, tends to return to the predetermined configuration when stretched or deformed from the predetermined configuration. The intragastric member 11 can be subject to stretching or deformation, such as, during deployment. Examples of shape memory polymers that may be used include polyurethanes, polynorborenes, styrene-butadiene co-polymers, cross-linked polyethylenes, cross-linked polycyclooctenes, polyethers, polyacrylates, polyamides, polysiloxanes, polyether amides, polyether esters, and urethane-butadiene co-polymers, and combinations thereof.

**FIG. 1** depicts a single intragastric member **11** in which the intragastric member **11** comprises a proximal end **13** and a distal end **14,** wherein the intragastric member **11** comprises a spiral coil **15.** The intragastric member **11** also comprises openings **16** positioned along the proximal end **13** and the distal end **14** of the intragastric member **11.** The openings **16** receive a one way valve utilized to inject or inflate pressurized gas and liquid into the lumen of the intragastric member **11,** thereby expanding the intragastric member **11** to a second configuration. Alternatively, the intragastric member **11** can comprise self expanding material, such as nitinol. The intragastric member **11** can also comprise one or more elongate portions inflatable from a first configuration to a second configuration, wherein the elongate portions comprise a lumen extending through a portion thereof. The lumen is inflated with a material to provide rigidity to the overall intragastric member **11.**

In a preferred embodiment, the intragastric member **11** comprises digestive-resistant or indigestible member **12** composed of a low density polyethylene. Fluorinated ethylene propylene, ethylene vinyl acetate copolymer, nylon, or types of polymers that are biocompatible and to which food will generally not adhere may also be utilized. The intragastric member **11** is available in a variety of material, sizes, shapes and diameters, which result in varying designs and configurations during advancement and placement in the stomach **60.**

Deployment of the intragastric member **11** can be accomplished in a number of ways, depending on the size, number and configuration of the embodiments. In order to create an obesity treatment apparatus **10** that will be retained in the stomach **60,** it may be necessary to couple more than one intragastric member **11** together to form a grouping or set **45** of intragastric members. **FIG. 2** shows two intragastric members **11** that each have a coupling mechanism **26** (e.g., tether **27**) attached about them such that they can be drawn together and deployed to the gastric lumen. A push member **29,** such as a catheter or corrugated metal tube, is advanced into gastric lumen by using an endoscope, and is guided over the tethers **27** to urge a securing element **28,** such as a rubber patch, tightly against the two intragastric members **11.** The tethers **27** can then be cut, allowing the grouping **45** to float free within the stomach. This method can also be used to join additional intragastric members **11** to form a larger grouping **45.** Any practical number of intragastric members **11** can be joined in the manner described above, or delivered singly or in pairs, and then grouped together after all of the intragastric members **11** have been delivered to the lumen.

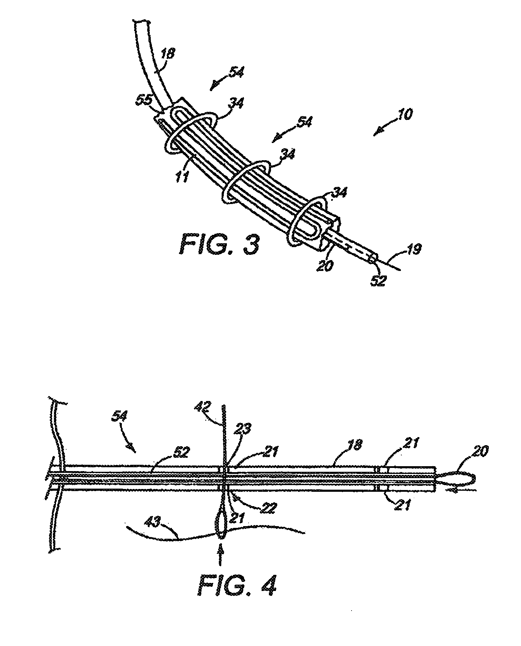

**FIG. 3** depicts a delivery system **54** in which the intragastric member **11** is mounted over a plastic overtube **18,** compressed by a sheath **55** and secured by retaining elements **34.** In particular, the intragastric member **11** is loaded over the overtube **18** and secured by the sheath **55,** which may be formed from a thin plastic material. In the illustrative embodiment, the retaining elements **34** or wire are looped under and over the sheath **55,** such that they can be withdrawn to tear through the thin material of the sheath **55** to release the intragastric member **11** mounted on the overtube **18.** A releasing mechanism **20** feeds into a passageway **52** of the overtube **18,** where it extends to the proximal end of the apparatus **10.** Other types of splittable sheaths **55** can also be used, such as the COOK® PEEL-AWAY Introducer Sheath available from Cook Inc., Bloomington, Indiana. A wire guide **19** is typically used during the delivery procedure, and is placed through the passageway of the overtube **18** to guide the distal end of the overtube **18** into the stomach of the patient.

As shown in **FIG. 4****,** the overtube **18** includes a plurality of apertures **21,** a pair of which (e.g., apertures **22** and **23**) are spaced apart a predetermined distance. Preferably, the apertures **22** and **23** are spaced apart approximately 2 cm along the distal portion of the overtube **18.** The apertures **22** and **23** may also be spaced apart by other distances.. To secure the intragastric member **11,** the retaining elements **34** are pulled through the first aperture **22** using a device **42** such as a loop, hook, snare, etc. It is fed through a releasing mechanism **20,** such as the illustrative wire loop, and then pulled through the opposite aperture **23.** The intragastric member **11** is then placed on the overtube **18,** and the retaining elements **34** are secured, thereby constraining the intragastric members **11** into a first configuration for delivery. Once the delivery system **54** has been introduced into the gastric lumen, the releasing mechanism **20** is pulled back through the overtube **18,** thereby severing the retaining elements **34,** one by one, and releasing the intragastric member **11** into the gastric lumen where it can assume a second configuration that is sufficiently voluminous such that they cannot pass from the stomach.

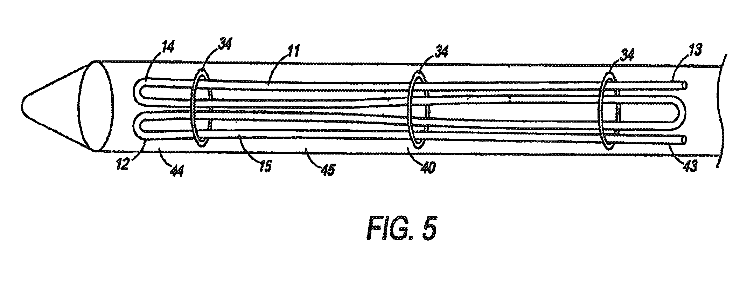

**FIG. 5** depicts a delivery tube **40** for delivering the intragastric member **11** of the present invention. The delivery tube **40** includes a proximal end **43,** a distal end **44** and a lumen **45,** wherein the intragastric member **11** is loaded onto the lumen **45** of the delivery tube **40** and secured by retaining elements **34.** The retaining elements **34** secure the intragastric member **11** along the lumen **45** of the delivery tube **40** from the distal end **44** to the proximal end **43** of the apparatus **10.** The number of retaining elements **34** needed depends on the size, length and width of the particular intragastric member **11** used in the apparatus **10.**

In the illustrative embodiment, the retaining elements **34** (see **FIG. 5**) are located equidistant about the body of the delivery tube **45** to secure the intragastric member **11.** However one of ordinary skill in the art would appreciate that other designs utilizing differently placed retaining elements **34,** or eliminating them entirely, could also be utilized.

Results from human trials may lead to modifications in the configuration being depicted in the figures of this application. Nevertheless, it is already understood that the dimensions shape, and construction of the intragastric member **11** can be quite variable and still produce the desired results.

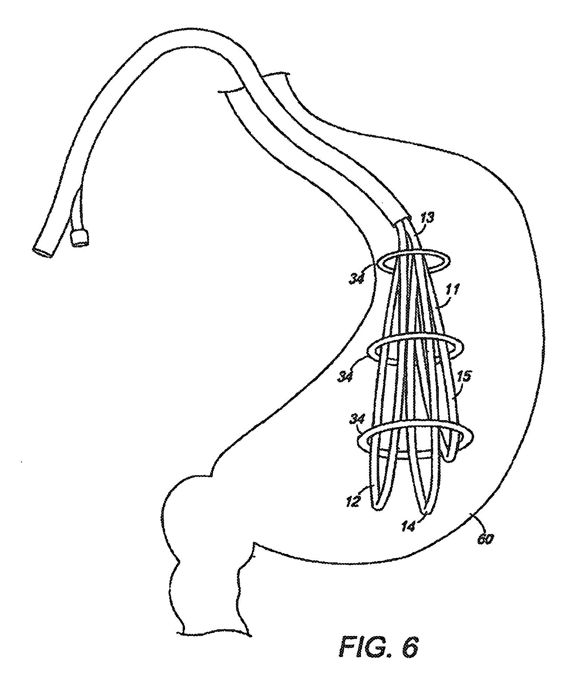

As illustrated in **FIGS. 6-21****,** varying shapes can be employed to increase the amount of space occupied by or vary the outer perimeter of the intragastric member. Particularly, the varying shapes can provide a feeling of fullness upon engaging in the lumen of the patient. The varying configurations of the intragastric member further provide complimentary designs that engage each other to displace volume after placement into the gastric lumen of the patient. It should be appreciated that other designs utilizing different diameters could also be utilized. The intragastric member can be composed of an expandable material, a low density polyethylene or other suitable material. The intragastric member is not limited to one particular shape, but can comprise varying shapes depending on the particular use. The shapes of the constituent components can be selected from the group consisting of spiral, circular, round, elliptical, square, triangular, rectangular, pentagonal, hexagonal, star-shaped or any other suitable shape.

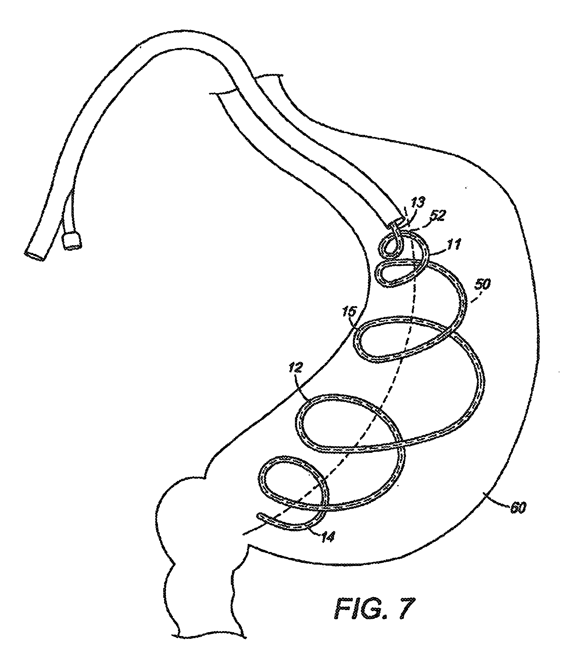

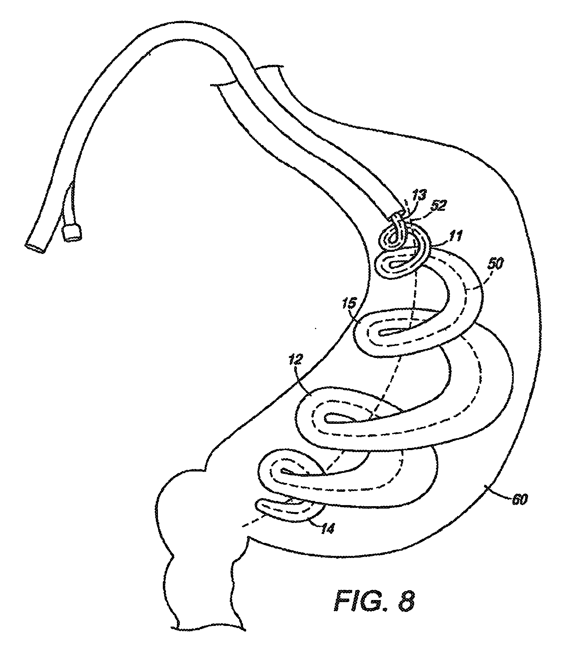

**FIGS. 6-8** depict an intragastric member **11** of the present invention expanding from a first configuration to a second configuration after delivery to the gastric lumen. The intragastric member **11** is coupled with the retaining elements **34** until delivered into the gastric lumen **(****FIG. 6****).** The retaining elements **34** are then removed from the intragastric member **11** and the intragastric member **11** self-expands to a second configuration **(****FIG. 7****).** In the alternative, the intragastric member **11** can be inflated via pressurized gas or liquid. In this embodiment, the intragastric member **11** comprises a self-expanding material, such as nitinol, to expand the intragastric member **11** to a second configuration wherein the intragastric member **11** is inflated and conforms to the interior contour of the stomach 60 and maintains contact with the wall of the stomach 60 **(****FIG. 8****).**

Additionally, the device 10 provides a central axis 52 and the intragastric member **11** comprises a curvilinear axis 50 which extends about and along the central axis 52 of the device 10. The term "central axis" as used herein is generally defined as a line extending along a major axis of the device (i.e., the device's longest dimension) and through the centroid of the device's general cross-section. The term "curvilinear axis" as used herein is generally defined as extending along the length of the intragastric member **11** and through the intragastric member's **11** cross-section. The curvilinear axis 50 of the intragastric member **11** is spaced away from the central axis 52 by a predetermined distance or a variable distance. The intragastric member **11** can form a shape comprising one of a spiral, helix, coil, cork screw, spring and loop. In this embodiment, the preformed spiral coil 15 of the intragastric member **11** forms a longitudinal configuration with the wall of the stomach **60.**

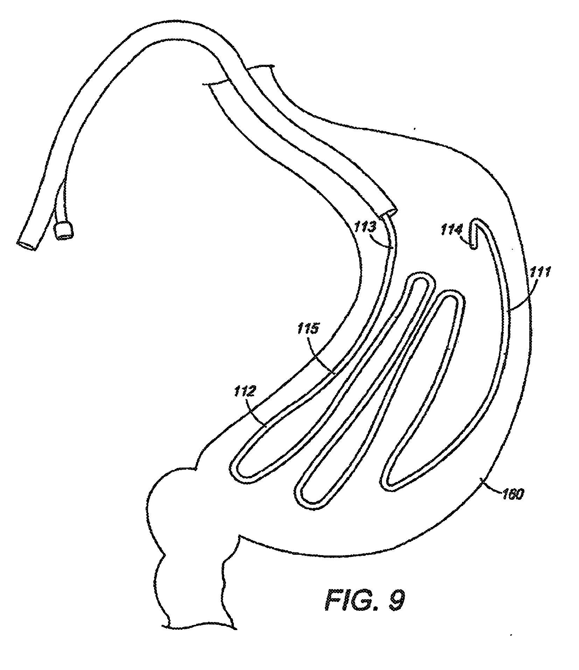

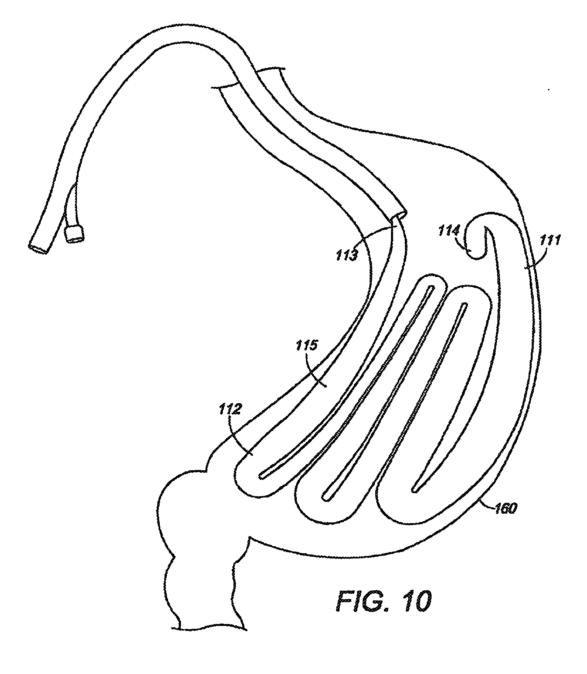

**FIGS. 9-10** depict an alternative embodiment of the intragastric member, wherein the intragastric member **111** comprises a preformed spiral coil **15** forming a latitudinal configuration with the wall of the stomach **160.** Similar to the longitudinal configuration, the intragastric member **111** comprises a proximal end **113,** a distal end **114** and a spiral coil **115.** Additionally, the intragastric member **111** can include an indigestible member **112** composed of a low density polyethylene. The intragastric member **111** can be inflated via pressurized gas or liquid **(****FIG.** 10), or include a self-expanding material **(****FIG. 9****).**

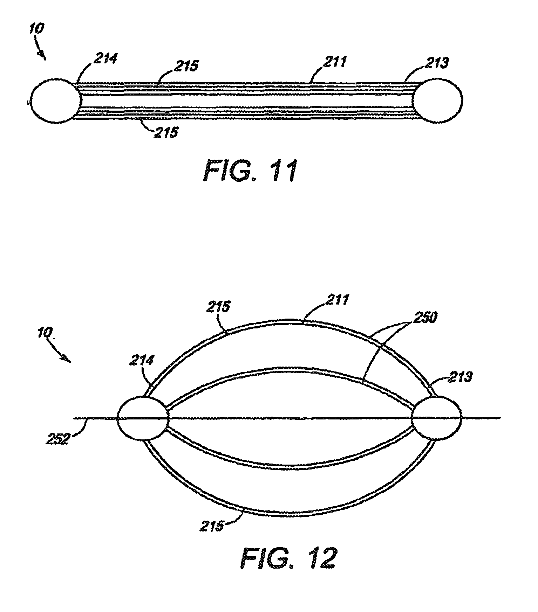

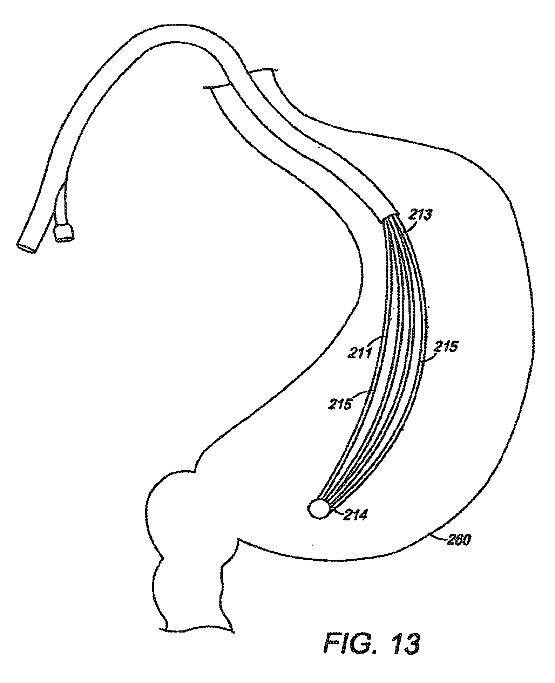

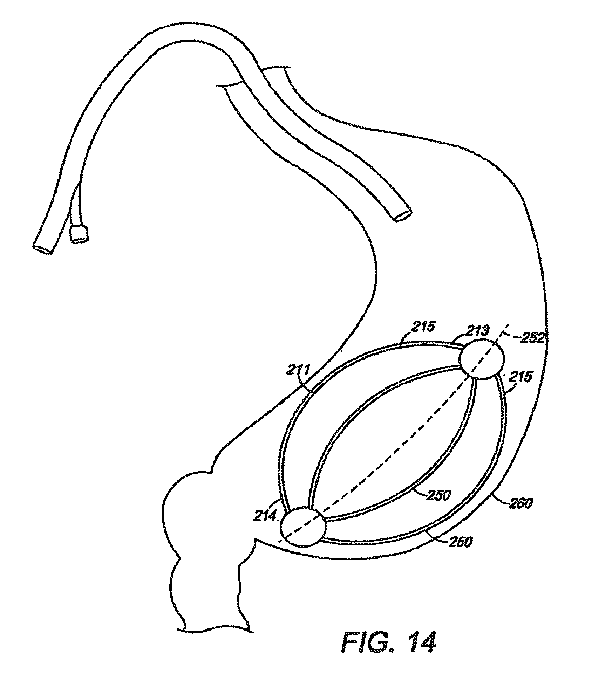

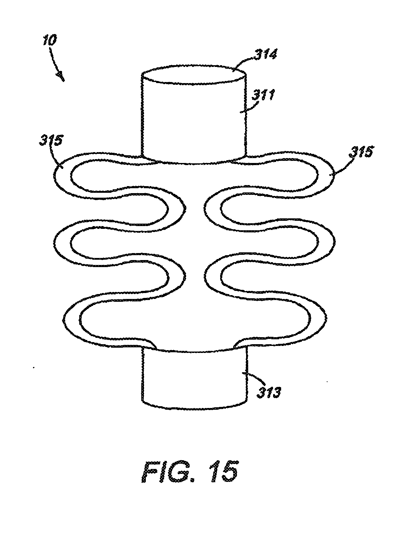

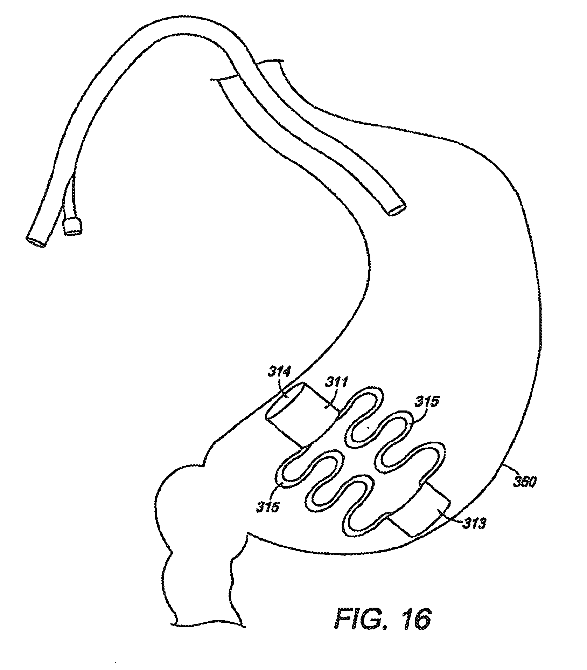

**FIG. 11-14** depicts yet another embodiment of an intragastric member **211** of the present invention. In this embodiment, the intragastric member **211** comprises a plurality of ribs **215** composed of a self-expanding material, such as nitinol, that has been compacted in a first configuration for delivery **(****FIG. 11****).** The ribs **215** of the intragastric member **211** are aligned longitudinally in the first configuration during deployment into the stomach 260, where it subsequently expands into the second configuration **(****FIG. 12****).** The intragastric member **211** includes a proximal end **213** and distal end **214** wherein the distal end **214** is passed into the gastric lumen during delivery. The intragastric member **211** is delivered in a first configuration with or without a catheter-based delivery system **54,** depending on the outer dimensions of the apparatus **10** (**FIG. 13**). The intragastric member **211** is expanded in the gastric lumen of the stomach **260** as the intragastric member **211** is delivered to the gastric lumen, wherein the ribs **215** engage the walls of the stomach **260 (****FIG. 14****).** Alternatively, the intragastric member **211** may be coated with a polymer or other suitable material to facilitate delivery and preservation of the intragastric member **211** in the gastric lumen. The intragastric member can also include other shapes and designs, such as circular, rectangular, hexagonal, elliptical or any other suitable shape. For example, **FIGS. 15**-16 depict another embodiment of an intragastric member **311** of the present invention, wherein the intragastric member **311** comprises a proximal end **313** and a distal end **314,** wherein a plurality of ribs **315** extend between the proximal end **313** and a distal end **314 (****FIG. 15****).** The configuration of the intragastric member **311** allows the corresponding ribs **315** to be compressed between the proximal end **313** and the distal end **314** during delivery. Both the proximal end **313** and the distal end **314** of the intragastric member **311** engage the wall of the stomach **360** after delivery and subsequent expansion to a second configuration **(****FIG. 16****).** The intragastric member **311** comprises two ribs **315.** However, other designs can include additional ribs **315.** The intragastric member **311** can be engaged longitudinally or latitudinally against the stomach wall depending on the configuration of the apparatus **10.**

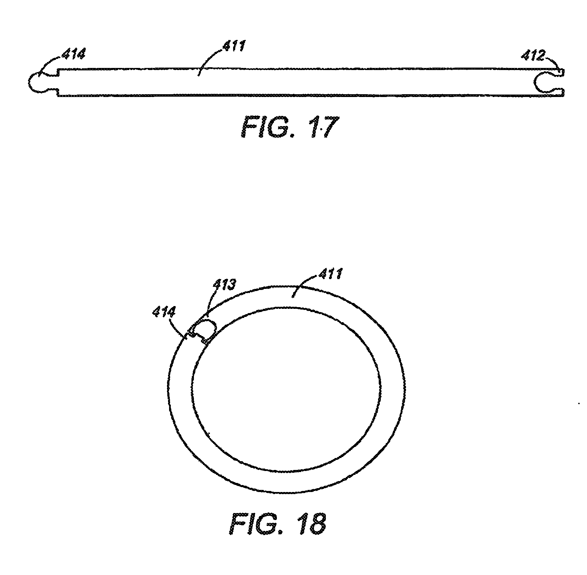

**FIGS. 17-21** depict yet another embodiment of an intragastric member **411** of the present invention. In this embodiment, the intragastric member **411** comprises a proximal end **413** and a distal end **414,** wherein the proximal end **413** includes a female locking component and the distal end **414** includes a male locking component of a locking mechanism **(****FIG. 17****).** The locking mechanism is utilized to connect the proximal end **413** and the distal end **414** of the intragastric member **411** to thereby form a band **(****FIG. 18****).**

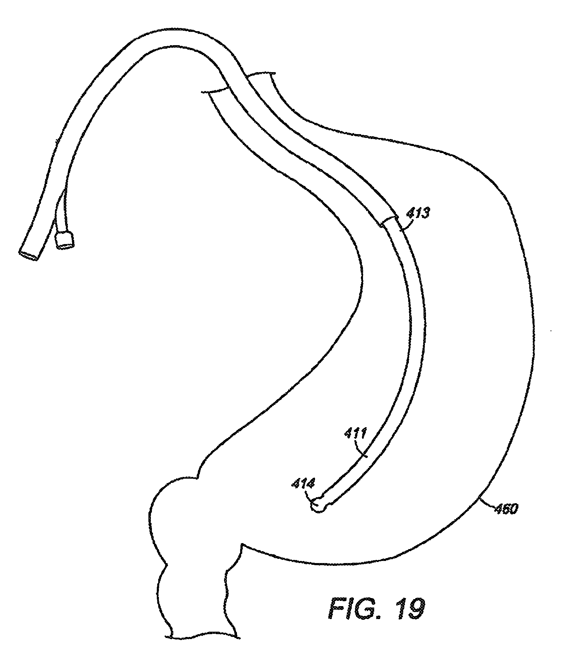

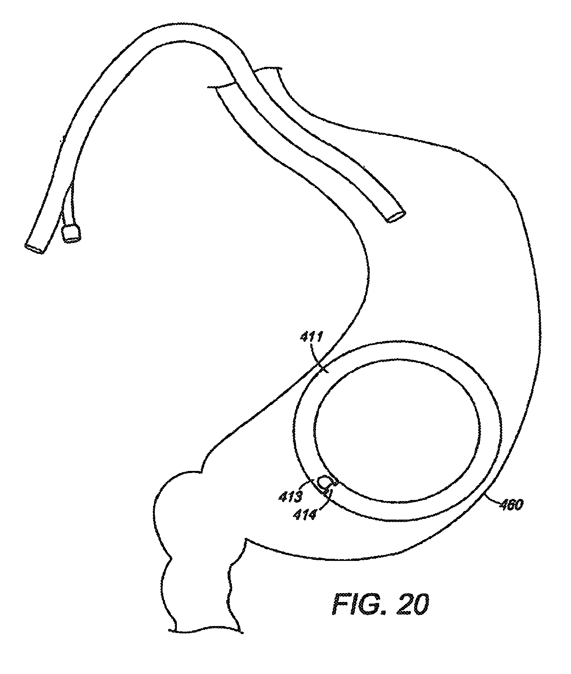

The intragastric member **411** is delivered to the gastric lumen in a first configuration, as shown in FIG. 19. The intragastric member **411** is delivered in a first configuration in which the proximal end 413 and the distal end **414** remain unconnected. Upon delivery into the gastric lumen, the intragastric member **411** is expanded to a second configuration wherein the proximal end of the intragastric member is connected to the distal end to form a band, wherein the band engages the wall of the stomach **460 (****FIG. 20****).** As depicted in **FIG. 20****,** the intragastric member **411** is delivered to the gastric lumen in a first configuration.

The illustrative embodiments of intragastric members **11, 111, 211, 311, 411** can be delivered in a number of ways, depending on the size, number, and configuration of the devices, or according to the physician's preference. Likewise, the intragastric members can be joined together, or they can be delivered singly or in pairs, and grouped together after all the intragastric members have been placed.

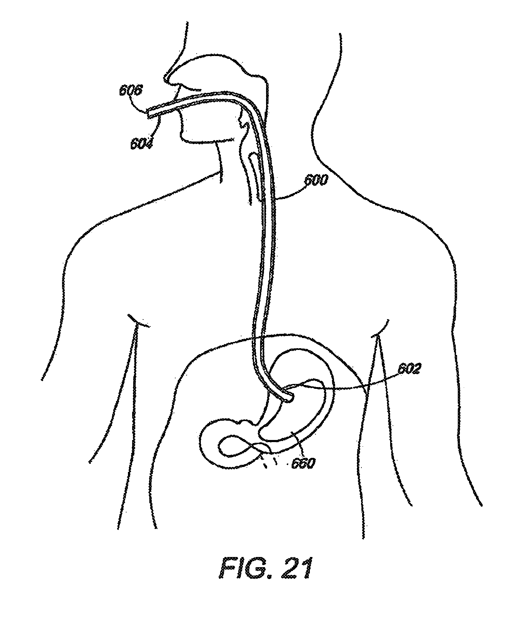

FIG. 21 depicts an overtube **600** that is used to deliver an intragastric member to the gastric lumen of the patient. The overtube **600** is used in combination with an endoscope to establish a passageway to a target delivery site in the stomach. Once the overtube **600** is positioned in the gastric lumen of the patient, the intragastric member is passed through the overtube **600,** and is used to deliver the intragastric member to the stomach **660** of the patient. Once the desired delivery in the gastric lumen is complete, the overtube **600** is removed.

The overtube **600** comprises a proximal end **604,** a distal end **602** and a main lumen 606. Any arrangement of the main lumen **606** is contemplated. The flexible overtube **600** can have a single-piece construction as shown in the embodiment depicted in **Figure 21****.** Alternatively, several tubes may be bonded together to form the flexible overtube **600** (not shown). The overtube **600** can be made from any suitable material known in the art including, but not limited to, polyethylene ether ketone (PEEK), polytetrafluorethylene (PTFE), polyamide, polyurethane, polyethylene and nylon, including multi-layer or single layer structures and may also include reinforcement wires, braid wires, coils and or filaments.

The main lumen **606** is configured to receive and pass an intragastric member, or suitable secondary device, such as an endoscope. The main lumen **606** ranges in size depending on the size of the intragastric member deployed. The size of the overtube **600** and corresponding intragastric member is provided for illustrative purposes only and are not intended to be construed as a limitation of the present invention. As one of ordinary skill in the art would appreciate, since the intragastric member and the endoscope and are advanced through the main lumen **606,** the size of the main lumen **606** is related to the size of either the intragastric member or the endoscope, which ever is larger. One of ordinary skill in the art would also appreciate that the size of the intragastric member is related to the length, width, and material comprising the intragastric member. Thus, a flexible overtube **600** may have smaller or larger dimensions depending on the size of the intragastric member, endoscope or other secondary device used in conjunction with the overtube **600** and therefore any overtube **600** of varying dimensions is contemplated as being within the scope of the claims of the present invention.

Having described the structures of the various intragastric members and delivery devices, a method of treatment of obesity in mammals will now be discussed. One type of method will now be described. An overtube 600 (FIG. 21) is positioned in the gastric lumen of the patient. After positioning the overtube 600 as shown in FIG. 21, at least one intragastric member 11 (FIG. 1) is loaded into a lumen 45 between a proximal end and distal end of a delivery tube 40 (FIG. 5). The intragastric member 11 is secured along the lumen 45 of the delivery tube 40 by retaining elements 34 (FIG. 5). The intragastric member 11 may comprise a preformed spiral coil or other suitable shape compacted into a first configuration that is sufficiently small to permit introduction into the gastric lumen of mammal.

After loading the at least one intragastric member 11 into the lumen 45 of the delivery tube 40, the delivery tube 40 is advanced through the overtube 600 until a distal end of the delivery tube 40 is positioned in the gastric lumen. The intragastric member 11 remains coupled with the retaining elements 34. After the delivery tube 40 has been positioned in the gastric lumen, the retaining elements 34 are removed from the intragastric member 11, thereby allowing the intragastric member 11 to self-expand to a second configuration (FIG. 7). Alternatively, the intragastric member 11 may be inflated through a lumen of the intragastric member 11 to conform to the interior contour of the stomach 60 (FIG. 8). The second configuration comprises a preformed spiral coil that is sufficiently large to prevent the intragastric member from passing through the mammal's pylorus.

Any other undisclosed or incidental details of the construction or composition of the various elements of the disclosed embodiment of the present invention are not believed to be critical to the achievement of the advantages of the present invention, so long as the elements possess the attributes needed for them to perform as disclosed. The selection of these and other details of construction are believed to be well within the ability of one of even rudimentary skills in this area, in view of the present disclosure. Illustrative embodiments of the present invention have been described in considerable detail for the purpose of disclosing a practical, operative structure whereby the invention may be practiced advantageously. The designs described herein are intended to be exemplary only. The novel characteristics of the invention may be incorporated in other structural forms without departing from the scope of the invention.

## Claims

1. An intragastric device for the treatment of obesity, the intragastric device comprising:
an intragastric member expandable from a first configuration to a second configuration, the first configuration being sufficiency small to permit introduction of said intragastric member into a gastric lumen of a mammal, the second configuration being sufficiently large to prevent said intragastric device from passing through the mammal's pylorus; and **characterised in that**
the intragastric device comprises a central axis, extending along a major axis of the device and wherein the intragastric member in the second configuration comprises a curvilinear axis which extends along the length of the intragastric member and through the intragastric member's cross section, the curvilinear axis extending circumferentially about and along the central axis of the intragastric device, the curvilinear axis being spaced away from the central axis by a variable distance.

2. The intragastric device according to claim 1, wherein the distance between the curvilinear axis and the central axis is greater at a midpoint of the intragastric device than at either end of the intragastric device.

3. The intragastric device according to any preceding claim, wherein the intragastric member comprises one of a spiral, helix, coil, cork screw, spring, preformed coil, and loop.

4. The intragastric device according to claim 1, wherein the intragastric member comprises a proximal end, a distal end and a lumen extending between the proximal end and the distal end of the intragastric member, wherein the lumen is utilized to inflate the intragastric member to the second configuration.

5. The intragastric device according to claim 4 further comprising an opening in communication with the lumen, wherein the opening is utilized to inflate the intragastric member.

6. The intragastric device according to one of claims 4 and 5, wherein the intragastric member is suited to be inflated with pressurized gas or liquid.

7. The intragastric device according to any preceding claim, further comprising a plurality of intragastric members secured with a releasing mechanism, wherein said plurality of intragastric members are secured in the first configuration.

8. The intragastric member according to claim 7, wherein the plurality of intragastric members are loaded through a delivery tube, wherein the delivery tube facilitates the delivery of each of the plurality of intragastric members from the delivery tube into the gastric lumen.

9. The intragastric device according to any preceding claim, further comprising an overtube comprising a proximal end, a distal end and a lumen configured to receive the intragastric member in the first configuration for delivery to the gastric lumen, wherein the intragastric member is expanded to the second configuration when in the gastric lumen.

10. The intragastric device according to any preceding claim, wherein said intragastric member comprises one or more elements selected from the group consisting of plastic, nylon, polyesters, polyurethanes, polyethylenes, polyamides, silicone and biocompatible polymers to which food will generally not adhere.

11. The intragastric device according to one of claims 1-9, wherein said intragastric member comprises one or more elements selected from the group consisting of high-density polyethylene, low-density polyethylene, fluorinated ethylene propylene and ethylene vinyl acetate copolymer.

12. The intragastric device according any preceding claim, wherein the intragastric member comprises a self-expanding metal or a shape memory plastic.

## Patentansprüche

1. Intragastrische Vorrichtung zur Behandlung von Fettsucht, wobei die intragastrische Vorrichtung Folgendes umfasst:
ein intragastrisches Element, das von einer ersten Konfiguration in eine zweite Konfiguration ausgefahren werden kann, wobei die erste Konfiguration ausreichend klein ist, um die Einführung des intragastrischen Elements in ein Magenlumen eines Säugetiers zu gestatten, wobei die zweite Konfiguration ausreichend groß ist, um zu verhindern, dass die intragastrische Vorrichtung durch den Pylorus des Säugetiers gleitet; und **dadurch gekennzeichnet, dass** die intragastrische Vorrichtung eine sich über eine Hauptachse der Vorrichtung erstreckende Mittelachse umfasst und worin das intragastrische Element in der zweiten Konfiguration eine krummlinige Achse umfasst, die sich über die Länge des intragastrischen Elements und durch den Querschnitt des intragastrischen Elements erstreckt, wobei die krummlinige Achse sich um den Umfang und entlang der Mittelachse der intragastrischen Vorrichtung erstreckt, wobei die krummlinige Achse über einen variablen Abstand von der Mittelachse beabstandet ist.

2. Intragastrische Vorrichtung nach Anspruch 1, worin der Abstand zwischen der krummlinigen Achse und der Mittelachse an einem Mittelpunkt der intragastrischen Vorrichtung größer ist als an einem der beiden Enden der intragastrischen Vorrichtung.

3. Intragastrische Vorrichtung nach einem der vorhergehenden Ansprüche, worin das intragastrische Element eine Spirale, eine Helix, eine Spule, einen Korkenzieher, eine Feder, eine vorgeformte Spule oder eine Schlaufe umfasst.

4. Intragastrische Vorrichtung nach Anspruch 1, worin das intragastrische Element ein proximales Ende, ein distales Ende und ein sich zwischen dem proximalen Ende und dem distalen Ende des intragastrischen Elements erstreckendes Lumen umfasst, worin das Lumen zum Aufblasen des intragastrischen Elements in die zweite Konfiguration verwendet wird.

5. Intragastrische Vorrichtung nach Anspruch 4, die ferner eine mit dem Lumen in Verbindung stehende Öffnung umfasst, worin die Öffnung zum Aufblasen des intragastrischen Elements verwendet wird.

6. Intragastrische Vorrichtung nach einem der Ansprüche 4 und 5, worin das intragastrische Element mit unter Druck stehendem Gas oder einer Druckflüssigkeit aufgeblasen werden kann.

7. Intragastrische Vorrichtung nach einem der vorhergehenden Ansprüche, die ferner eine Vielzahl von intragastrischen Elementen umfasst, die mit einem Lösemechanismus fixiert sind, worin die Vielzahl von intragastrischen Elementen in der ersten Konfiguration fixiert sind.

8. Intragastrische Vorrichtung nach Anspruch 7, worin die Vielzahl von intragastrischen Elementen durch ein Abgaberohr geladen werden, worin das Abgaberohr die Abgabe jedes der Vielzahl von intragastrischen Elementen aus dem Abgaberohr in das Magenlumen erleichtert.

9. Intragastrische Vorrichtung nach einem der vorhergehenden Ansprüche, die ferner einen Schutzschlauch mit einem proximalen Ende, einem distalen Ende und einem zur Aufnahme des intragastrischen Elements in der ersten Konfiguration zur Abgabe an das Magenlumen konfiguriertes Lumen umfasst, worin das intragastrische Element in die zweite Konfiguration aufgeweitet wird, wenn es sich im Magenlumen befindet.

10. Intragastrische Vorrichtung nach einem der vorhergehenden Ansprüche, worin das intragastrische Element ein oder mehr Elemente umfasst, die aus der aus Kunststoff, Nylon, Polyestern, Polyurethanen, Polyethylenen, Polyamiden, Silikon und biokompatiblen Polymeren, an denen im Allgemeinen keine Nahrung anhaftet, bestehenden Gruppe ausgewählt sind.

11. Intragastrische Vorrichtung nach einem der Ansprüche 1-9, worin das intragastrische Element ein oder mehr Elemente umfasst, die aus der aus Polyethylen hoher Dichte, Polyethylen niederer Dichte, fluoriertem Ethylenpropylen und Ethylen-Vinylacetat-Copolymer bestehenden Gruppe ausgewählt sind.

12. Intragastrische Vorrichtung nach einem der vorhergehenden Ansprüche, worin das intragastrische Element ein selbstexpandierendes Metall oder einen Formgedächtniskunststoff umfasst.

## Revendications

1. Dispositif intragastrique pour le traitement de l'obésité, le dispositif intragastrique comportant :
un organe intragastrique déployable d'une première configuration a une deuxième configuration, la première configuration étant suffisamment petite pour permettre l'introduction dudit organe intragastrique dans une lumière gastrique de mammifère, la deuxième configuration étant suffisamment grande pour empêcher ledit dispositif intragastrique de traverser le pylore du mammifère ; et **caractérisé en ce que**
le dispositif intragastrique comporte un axe central s'étendant le long d'un axe principal du dispositif, et dans lequel l'organe intragastrique dans la deuxième configuration comporte un axe curviligne qui s'étend le long de l'organe intragastrique et dans la section transversale du dispositif intragastrique, l'axe curviligne s'étendant de manière circonférentielle autour de l'axe central du dispositif intragastrique et le long de celui-ci, l'axe curviligne étant éloigné de l'axe central par une distance variable.

2. Dispositif intragastrique selon la revendication 1, dans lequel la distance entre l'axe curviligne et l'axe central est plus grande au niveau d'un point médian de l'organe intragastrique que l'une ou l'autre des extrémités du dispositif intragastrique.

3. Dispositif intragastrique selon l'une quelconque des revendications précédentes, dans lequel l'organe intragastrique comporte une spirale, une hélice, un serpentin, un tire-bouchon, un ressort, un serpentin préformé, et une boucle.

4. Dispositif intragastrique selon la revendication 1, dans lequel l'organe intragastrique comporte une extrémité proximale, une extrémité distale et une lumière qui s'étend entre l'extrémité proximale et l'extrémité distale de l'organe intragastrique, la lumière étant utilisée pour le gonflage de l'organe intragastrique dans la deuxième configuration.

5. Dispositif intragastrique selon la revendication 4 comportant en outre une ouverture communiquant avec la lumière, dans lequel l'ouverture est utilisée pour le gonflage de l'organe intragastrique.

6. Dispositif intragastrique selon l'une quelconque des revendications 4 et 5, dans lequel l'organe intragastrique est adapté pour être gonflé avec du gaz ou liquide pressurisé.

7. Dispositif intragastrique selon l'une quelconque des revendications précédentes, comportant en outre plusieurs organes intragastriques fixés au moyen d'un mécanisme de libération, dans lequel lesdits plusieurs organes intragastrique sont fixés dans la première configuration.

8. Organe intragastrique selon la revendication 7, dans lequel lesdits plusieurs organes intragastriques sont chargés par un tube de mise en place, le tube de mise en place facilitant la mise en place de chaque organe intragastrique desdits plusieurs organes intragastriques depuis le tube de mise en place dans la lumière gastrique.

9. Dispositif intragastrique selon l'une quelconque des revendications précédentes, comportant en outre un surtube comportant une extrémité proximale, une extrémité distale et une lumière, configuré pour recevoir l'organe intragastrique dans la première configuration afin de le mettre en place dans la lumière gastrique, l'organe intragastrique étant déployé dans la deuxième configuration lorsqu'il est dans la lumière gastrique.

10. Dispositif intragastrique selon l'une quelconque des revendications précédentes, dans lequel l'organe intragastrique comporte un ou plusieurs éléments choisis dans le groupe constitué par la matière plastique, le nylon, les polyesters, les polyuréthannes, les polyéthylènes, les polyamides, la silicone et les polymères biocompatibles auxquels les aliments en général n'adhèrent pas.

11. Dispositif intragastrique selon l'une quelconque des revendications 1 à 9, dans lequel ledit organe intragastrique comporte un ou plusieurs éléments choisis dans le groupe constitué par le polyéthylène de haute densité, le polyéthylène de faible densité, l'éthylène-propylène fluoré et un copolymère d'éthylène-vinyl-acétate.

12. Dispositif intragastrique selon l'une quelconque des revendications précédentes, dans lequel l'organe intragastrique comporte un métal autodéployable ou un plastique à mémoire de forme.
